# EUROPÄISCHE PATENTSCHRIFT

(11) **EP 4 346 709 B1**
(45) Veröffentlichungstag und Bekanntmachung des Hinweises auf die Patenterteilung: **06.08.2025**
(21) Anmeldenummer: 22728336.3
(22) Anmeldetag: 01.06.2022
(51) Int. Cl.: A61F 5/01, A61H 9/00, A61F 5/03

(54) **VORRICHTUNG ZUR NICHTINVASIVEN TRICHTERBRUSTKORREKTUR**
APPARATUS FOR NON-INVASIVE CORRECTION OF PECTUS EXCAVATUM
APPAREIL DE CORRECTION NON INVASIVE DE PECTUS EXCAVATUM

(30) Priorität: 02.06.2021 AT 504472021
(43) Veröffentlichungstag der Anmeldung: 10.04.2024
(73) Patentinhaber: Rokitansky, Alexander, 1190 Wien (AT)
(72) Erfinder: Rokitansky, Alexander, 1190 Wien (AT)
(74) Vertreter: SONN Patentanwälte GmbH & Co KG
(86) Internationale Anmeldenummer: PCT/AT2022/060184
(87) Internationale Veröffentlichungsnummer: WO 2022/251891

(56) Entgegenhaltungen:
- CN-U- 209 075 066
- CN-U- 211 213 735
- DE-U1- 20 301 235

## Beschreibung

Die Erfindung betrifft eine Vorrichtung zur nichtinvasiven Trichterbrustkorrektur, mit einer Saugglocke zum Aufsetzen auf den Brustkorb eines Patienten, wobei die Saugglocke einen Mittelteil und einen daran anschließenden, umlaufenden Auflagerand aus weichem Material zur Auflage auf dem Brustkorb sowie einen Anschluss zur Verbindung mit einer Luftpumpe aufweist.

Die Trichterbrust (Pectus excavatum) stellt die häufigste Fehlbildung der Thoraxwand dar, welche häufiger bei Buben als bei Mädchen auftritt und sich zumeist in der Pubertät entwickelt bzw. verstärkt. In einem Drittel der Fälle konnte ein familiärer Zusammenhang beobachtet werden. Bei älteren Patienten treten auch asymmetrische Trichterbildungen auf, wobei die Biegung nicht mehr nur im knorpeligen, sondern auch vom knöchernen Teil der Rippe vorliegt. Neben kosmetischen Aspekten der Fehlbildung wird vor allem der Druck auf das Herz und das damit verbundene Leistungslimit zum gesundheitlichen Problem.

Das therapeutische Management umfasst die physikalische Therapie ergänzt durch eine operative Korrektur. Dieses minimal invasive Verfahren, ohne Knorpelresektion, mit Implantation eines individuell geformten Bügels stellt in der Mehrzahl der Fälle eine Alternative mit vergleichbar ausgezeichneten Behandlungsresultaten dar. Durch diese sogenannte "Pectus Bar"-Implantation erfolgt eine endgültige Korrektur der Thoraxdeformität über einen Zeitraum von 2 bis 4 Jahren, wonach der implantierte Bügel wieder entfernt wird.

Beispielsweise beschreibt die AT 413 479 B ein Implantat für die Trichterbrustkorrektur mit einem Bügel und zumindest einer mit dem Bügel verbindbaren Stabilisatorplatte zur Befestigung des Implantats am Brustkorb.

Zur nichtinvasiven Behandlung werden Saugglocken angewendet, welche auf den Brustkorb aufgesetzt werden. Mithilfe einer Saugpumpe wird zwischen der Saugglocke und der Brust des Patienten ein Unterdruck erzeugt, wodurch eine Zugkraft auf die nach innen gewölbten Teile des Brustkorbs ausgeübt wird. Bei häufiger, insbesondere täglicher Anwendung über mehrere Stunden kann der Brustkorb in Richtung einer physiologischen Form verändert und eine Dehnung des Gewebes erzielt werden, der Druck auf das Herz gemindert bzw. aufgehoben werden, die Atemmechanik verbessert werden und der Trichter abgeflacht und die Ausgangslage für eine gegebenenfalls notwendige Operation verbessert und die Rekonvaleszenz verkürzt werden.

Die CN 209075066 U beschreibt eine Saugglocke zur nichtinvasiven Trichterbrustkorrektur der gegenständlichen Art mit einem im Wesentlichen elliptischen bzw. ovalen Mittelteil, der unten in der Mitte einen dreieckigen oder halbkreisförmigen Einschnitt aufweist. Weiters kann der Mittelteil im Bereich der Brustwarzen bzw. Brust seitliche Aussparungen aufweisen, um eine Kompression der Brustwarzen bzw. Brust zu vermeiden.

Die DE 197 34 571 B4 beschreibt eine solche Saugglocke zur nicht-chirurgischen Korrektur einer Trichterbrust, welche sich seit langer Zeit sehr bewährt hat. Die Saugglocke weist einen im Wesentlichen ovalen bzw. kreisförmigen symmetrischen Mittelteil auf und ist für Männer in vier und für Frauen in einer fixen Größe erhältlich.

Die CN 211213735 U zeigt eine Saugglocke zur Trichterbrustkorrektur mit einem im Wesentlichen ovalen Mittelteil, der zwei Einbuchtungen im Bereich der Brust aufweist, um eine Kompression der Brust bzw. Brustwarzen zu vermeiden.

Die CN 111759561 A beschreibt eine Vorrichtung zur nichtinvasiven Trichterbrustkorrektur, welche an die ungleichmäßigen Verformungen des Brustkorbes eines Patienten besser angepasst werden kann, indem zumindest ein Teil des Randes der Saugglocke in Form einer Faltenstruktur ausfahrbar ausgebildet ist. An jener Stelle des Randes der Saugglocke, an der die Faltenstruktur vorgesehen ist, kann die Höhe des Randes verändert und an die jeweiligen Gegebenheiten angepasst werden.

Der Einsatz der verwendeten Saugglocke ist auf symmetrische Trichterbrustformen beschränkt. Asymmetrische Formen, wo beispielsweise der Trichter auf eine Körperhälfte beschränkt ist, sind mit den angebotenen fixen Größen nicht oder nur unzureichend erfolgreich zu behandeln. Auch gemischte Formen (Kiel-/ Trichterdeformität), wo einerseits sowohl ein Vorwölbung am Brustkorb und an anderer Stelle eine Einsenkung vorliegt, sind mit herkömmlichen Saugglocken schwer zu behandeln. Der Trichter blieb bestehen und die Kielbildung kann sogar noch verstärkt werden.

Die Aufgabe der vorliegenden Erfindung besteht daher in der Schaffung einer oben genannten Vorrichtung, durch welche auch spezielle Trichterbrustformen und insbesondere asymmetrische Trichterbrustformen zugleich mit gegebenenfalls gleichzeitig bestehenden kielförmigen Vorwölbungen optimal therapeutisch behandelt werden können. Nachteile bekannter Vorrichtungen sollen vermieden oder zumindest reduziert werden.

Gelöst wird die erfindungsgemäße Aufgabe dadurch, dass der Mittelteil an seiner Unterseite in der Gebrauchslage der Saugglocke im Bereich der unteren Rippenbögen zumindest einen lateral und schräg nach unten weisenden Fortsatz aufweist, und dass der Auflagerand im Bereich des zumindest einen Fortsatzes eine Verbreiterung gegenüber der Breite des restlichen Auflagerandes aufweist, sodass ein Druck auf einen linken oder rechten unteren Rippenbogen ausübbar ist. Durch eine solche Abwandlung einer Saugglocke können spezielle Trichterbrustformen gegebenenfalls mit gleichzeitigen kielartigen Vorwölbungen, insbesondere asymmetrische Trichterbrustformen besser behandelt werden als dies mit herkömmlichen Vorrichtungen möglich war. Durch den zumindest einen lateral und schräg nach unten weisenden Fortsatz am Mittelteil der Saugglocke kann die Vorrichtung individuell an die jeweilige Form der Trichterbrust des Patienten oder der Patientin angepasst werden. Es entsteht im Wesentlichen die Form eines "J" oder gespiegelten "J", welche sich zur Therapie einer Trichterbrust mit zusätzlich kielartig vorspringendem linken oder rechten unteren Rippenbogen besonders eignet. Mit einer solchen Saugglocke wird einerseits der eingesunkene Bereich in der Mitte optimal angesaugt, während auf den vorspringenden deformierten linken oder rechten unteren Rippenbogen über den unter dem jeweiligen Fortsatz angeordneten Auflagerand während der Therapie ein Druck ausgeübt wird. Durch die spezielle Formgebung der Vorrichtung kann ein besserer therapeutischer Erfolg erzielt werden. Durch die spezielle Form des Mittelteils wird auf die nach innen gewölbten Teile des Thorax ein Sog bzw. eine Zugkraft ausgeübt, während zielgerichtet auf die atypisch vorgewölbten Stellen des Thorax zugleich eine Druckkraft ausgeübt wird. Somit herrscht in den Zonen der Einsenkung ein Sog bzw. Unterdruck und in den Zonen der Vorwölbung eine Kompression bzw. Überdruck, es findet also bei der Anwendung der Therapievorrichtung ein kombinierter Unter-/Überdruck auf den Thorax statt, wodurch schneller und wirkungsvoller ein positives Ergebnis verzeichnet werden kann. Im Bereich des zumindest einen Fortsatzes weist der Auflagerand eine entsprechend angepasste Verbreiterung auf. Da über den Auflagerand eine Druckkraft auf die darunterliegenden vorgewölbten Bereiche des Brustkorbes ausgeübt wird, wird durch eine entsprechende Verbreiterung die Kraftdichte exakt auf die vorgewölbten Bereiche positioniert. Die Verbreiterungen können beispielsweise 50% bis 200% der Breite des restlichen Auflagerandes betragen. Insbesondere bei gemischten Kiel-/Trichterdeformitäten ist diese Vorrichtung besonders vorteilhaft anwendbar. Die gegenständliche Vorrichtung kann relativ leicht und kostengünstig hergestellt werden. Einerseits kann der speziell gestaltete Mittelteil individuell an die Form der Trichterbrust angepasst werden, indem Magnetresonanz- oder Röntgenaufnahmen der Patienten zur genauen Vermessung herangezogen werden, oder in bestimmten Formen und Größen, welche den Großteil bekannter Trichterbrustformen abdecken, vorgefertigt werden. Vorzugsweise wird die Vorrichtung in drei verschiedenen Größen (klein, mittel, groß) für verschiedene Körpergrößen des Patienten oder der Patientin angefertigt. Die Länge des zumindest einen Fortsatzes beträgt einen Bruchteil der Höhe des Mittelteils, beispielsweise 20% bis 80% der Höhe des Mittelteils.

Vorteilhafterweise weist der Mittelteil an seiner Unterseite in der Gebrauchslage der Saugglocke im Bereich der unteren Rippenbögen beiderseits einen lateral und schräg nach unten weisenden Fortsatz auf, sodass ein Druck auf beidseitige untere Rippenbögen ausübbar ist. Dadurch weist der Mittelteil im Wesentlichen die Form eines umgekehrten "Y" auf, wobei die beiderseits angeordneten Fortsätze bei einer Trichterbrust mit zusätzlich beiderseits vorspringender unterer Rippenbögen optimal eingesetzt werden kann. Mit einer solchen Saugglocke wird einerseits der median oder leicht paramedian angeordnete eingesunkene Bereich optimal angesaugt, während auf die vorspringenden deformierten beidseitigen unteren Rippenbögen über den unter den Fortsätzen angeordneten Auflagerand ein Druck ausgeübt wird.

Der zumindest eine Fortsatz ist in einem Winkel von 25° bis 60° zu einer in der Gebrauchslage der Saugglocke vertikalen Richtung angeordnet. Derartige Neigungen des zumindest einen Fortsatzes haben sich bei bekannten Trichterbrustformen als besonders geeignet herausgestellt.

Der Mittelteil der Vorrichtung kann an seiner Oberseite in der Gebrauchslage der Saugglocke im oberen Brustkorbbereich zumindest eine seitliche Ausbuchtung aufweisen, sodass eingesunkene Bereiche im rechten und/oder linken Bereich des Brustkorb-Oberfeldes überdeckbar sind. Durch zumindest eine solche zusätzlich zu dem zumindest einen an der Unterseite des Mittelteils angeordneten Fortsatz vorgesehene seitliche Ausbuchtung kann optimal auf eingesunkene Bereiche im rechten oder linken oberen Brustkorbbereich reagiert werden. Die jeweilige rechts und/oder links an der Oberseite des Mittelteils angeordnete Ausbuchtung überdeckt in besonders geeigneter Weise den eingesunkenen Bereich im rechten und/oder linken Bereich des Burstkorb-Oberfeldes und bewirkt auch auf diese eingesunkenen Bereiche eine optimale Sogwirkung. Die Ansätze stehen im Wesentlichen quer vom Mittelteil, also im Wesentlichen 90° zur vertikalen Mittelachse des Mittelteiles nach rechts und/oder links ab. Die Längsausdehnung der seitlichen Ausbuchtung beträgt einen Bruchteil der Breite des Mittelteils, beispielsweise 20% bis 80% der Breite des Mittelteils.

Vorteilhafterweise weist der Auflagerand auch im Bereich der zumindest einen seitlichen Ausbuchtung eine entsprechend angepasste Verbreitung gegenüber der Breite des restlichen Auflagerandes auf. Da über den Auflagerand eine Druckkraft auf die darunterliegenden vorgewölbten Bereiche des Brustkorbes ausgeübt wird, wird durch eine entsprechende Verbreiterung die Kraftdichte exakt auf die vorgewölbten Bereiche positioniert. Die Verbreiterungen können beispielsweise 50% bis 200% der Breite des restlichen Auflagerandes betragen.

Der Anschluss zur Verbindung mit der Luftpumpe weist gemäß einem weiteren Merkmal der Erfindung ein Ventil auf. Dadurch kann die Luftpumpe, welche vorzugsweise durch eine einfache Handpumpe gebildet sein kann, nach Herstellung des Unterdrucks zwischen Saugglocke und Brustkorb abgenommen werden, ohne dass der Unterdruck reduziert wird. Danach kann die Vorrichtung ohne Luftpumpe beispielsweise auch tagsüber unter der Kleidung getragen werden, ohne dass dies besonders auffällt. Außerdem kann die Vorrichtung auch besser während sportlicher Aktivitäten getragen werden.

Am Mittelteil kann eine Vorrichtung zur Begrenzung des Unterdrucks zwischen Saugglocke und Brustkorb des Patienten bei der Anwendung der Saugglocke angeordnet sein, um zu verhindern, dass unzulässig hohe Kräfte auf den Brustkorb wirken und beispielsweise Hämatome unter den Auflagerändern entstehen. Diese Vorrichtung zur Begrenzung des Unterdrucks kann vorzugsweise durch ein Sicherheitsventil gebildet sein. Auch eine Kombination des oben erwähnten Ventils und Sicherheitsventils im Anschluss zur Verbindung mit der Luftpumpe ist denkbar.

Der Mittelteil kann im Wesentlichen eben ausgebildet sein. Eine ebene Gestaltung des Mittelteils ist besonders leicht herstellbar. Das Material und die Dicke des Mittelteils müssen so gewählt werden, dass sich der Mittelteil bei der Anwendung der Vorrichtung nicht wesentlich verformt und entsprechend stabil bleibt. Vorzugsweise wird ein geeigneter Kunststoff für die Herstellung des Mittelteils verwendet, beispielsweise Plexiglas mit einer Dicke von mehreren Millimetern.

Alternativ dazu kann der Mittelteil auch in Richtung Brustkorb gewölbt oder strukturiert ausgebildet sein. Durch eine derartige Wölbung oder Strukturierung kann bei geringerer Materialdicke eine höhere Stabilität erzielt werden und somit das Gewicht des Mittelteils und somit der gesamten Vorrichtung reduziert werden. Das reduzierte Gewicht wirkt sich wiederum positiv auf den Tragekomfort für den Patienten oder die Patientin aus.

Wenn der Mittelteil zumindest teilweise aus transparentem Material besteht, kann eine visuelle Beurteilung des Brustkorbs unter der Vorrichtung vorgenommen werden. Beispielsweise kann der gesamte Mittelteil aus transparentem Kunststoff, beispielsweise aus Plexiglas mit einer Dicke von mehreren Millimetern, gebildet sein.

Der Mittelteil und der Auflagerand kann gemeinsam hergestellt sein, vorzugsweise in einem Zweikomponenten-Spritzgussverfahren. Dadurch kann einerseits die Stabilität des Mittelteils und andererseits die höhere Elastizität des Auflagerandes in einem Herstellungsvorgang vereint werden. Beim Zwei- oder Mehrkomponenten-Spritzgießen können Teile aus zwei oder mehreren verschiedenen Kunststoffen hergestellt werden. Durch entsprechende Wahl der Kunststoffe können die gewünschten unterschiedlichen Eigenschaften der Teile der Vorrichtung gezielt kombiniert werden.

Der Mittelteil und der Auflagerand können aber auch getrennt hergestellt und vorzugsweise fest miteinander verbunden, beispielsweise miteinander verklebt oder verschweißt sein. Als Material für den Auflagerand eignet sich ein besonders elastisches Material, wie zum Beispiel Silikon, besonders.

Die vorliegende Erfindung wird anhand der beigefügten Figuren näher erläutert. Darin zeigen:
- Fig. 1: eine schematische Frontansicht auf eine Ausführungsform einer erfindungsgemäßen Vorrichtung zur nichtinvasiven Trichterbrustkorrektur bei der Anwendung;
- Fig. 2: einen horizontalen Schnitt durch die Vorrichtung gemäß Fig. 1 entlang der Schnittlinie II - II;
- Fig. 3O: und 3U schematische Ansichten auf eine weitere Ausführungsform einer Vorrichtung zur nichtinvasiven Trichterbrustkorrektur von oben und unten; und
- Fig. 4A: bis 4J verschiedene Ausführungsformen der Vorrichtung zur nichtinvasiven Trichterbrustkorrektur.

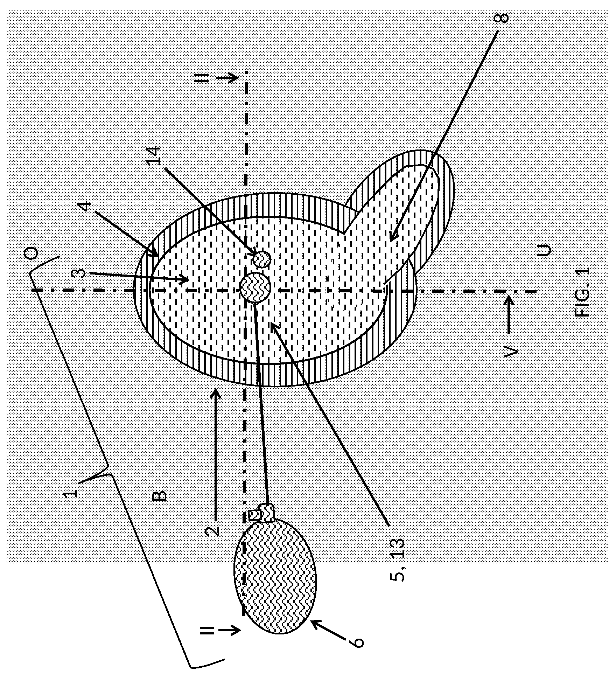

Fig. 1 zeigt eine schematische Frontansicht auf eine Ausführungsform einer erfindungsgemäßen Vorrichtung 1 zur nichtinvasiven Trichterbrustkorrektur bei der Anwendung am Brustkorb B eines Patienten P. Die Vorrichtung 1 beinhaltet eine Saugglocke 2 zum Aufsetzen auf den Brustkorb B. Die Saugglocke 2 weist einen Mittelteil 3 und einen daran anschließenden, umlaufenden Auflagerand 4 aus weichem Material auf, der auf den Brustkorb B aufgelegt wird. Am Mittelteil 3 ist ein Anschluss 5 zur Verbindung mit einer Luftpumpe 6 vorgesehen, mit der ein Unterdruck unter der Saugglocke 2 hergestellt werden kann. Vorzugsweise weist der Anschluss 5 ein Ventil 13 auf, wodurch die Luftpumpe 6 nach dem Absaugen der Luft aus der Saugglocke 2 abgenommen werden kann, ohne dass wieder Luft über den Anschluss 5 einströmen kann. Im Wesentlichen hält die Saugglocke durch den Unterdruck selbständig am Brustkorb B des Patienten P und kann unter der Kleidung über mehrere Stunden getragen werden.

Weiters kann am Mittelteil 3 eine Vorrichtung 14 zur Begrenzung des Unterdrucks zwischen Saugglocke 2 und Brustkorb B des Patienten P, vorzugsweise ein Sicherheitsventil, angeordnet sein. Beispielsweise kann der Unterdruck auf minus 0,3 bar limitiert werden. Wenn der Mittelteil 3 zumindest teilweise aus transparentem Material besteht, kann die Platzierung am Brustkorb B besser beurteilt werden.

Erfindungsgemäß weist der Mittelteil 3 an seiner Unterseite U in der Gebrauchslage der Saugglocke 2 zumindest einen lateral und gegenüber der vertikalen Richtung V schräg nach unten weisenden Fortsatz 8 auf, in der dargestellten Ausführungsform auf der rechten Seite bzw. linken Körperhälfte des Patienten P. Dadurch kann die Saugglocke 2 optimal und individuell auf die Trichterbrustform angepasst werden. Im dargestellten Beispiel weist der Mittelteil 3 der Saugglocke 2 die Form eines nach links weisenden "J" auf, welche Form speziell bei einer Trichterbrust mit zusätzlich vorspringendem linken unteren Rippenbogen geeignet ist.

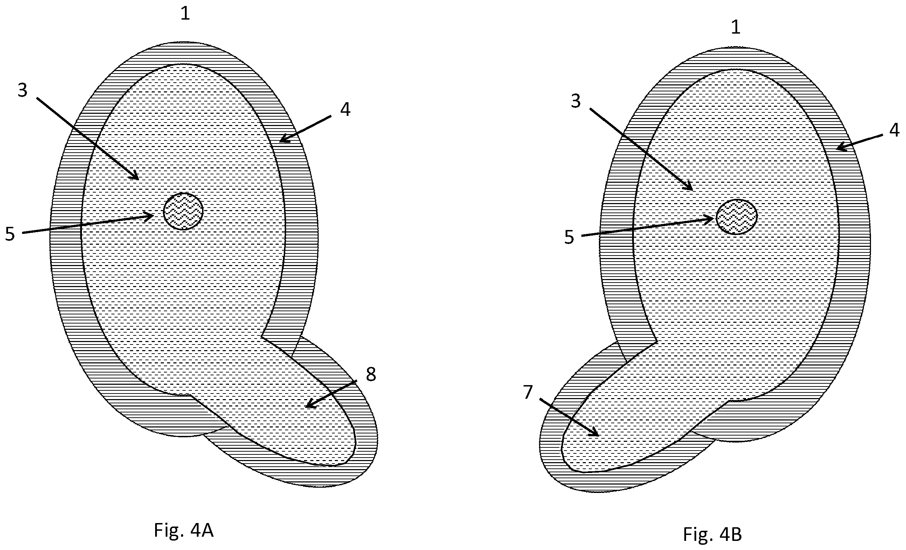

Natürlich können auch an beiden Seiten des Mittelteils 3 an der Unterseite U Fortsätze 7, 8 und auch an der Oberseite O des Mittelteils 3 seitliche Ausbuchtungen 11, 12 angeordnet sein, wie anhand der Ausführungsbeispiele der Figuren 4A bis 4J weiter unten veranschaulicht wird. Die Dimensionen des Mittelteils 3, des zumindest einen Fortsatzes 8 und der allfälligen Ausbuchtung 11 kann prinzipiell individuell an die jeweilige Trichterbrustform angepasst werden. Für die meisten Anwendungen können jedoch bestimmte Formen in unterschiedlichen Dimensionen (klein, mittel, groß) vorgefertigt werden und aus diesem Set an möglichen Saugglocken 2 die passendste Form ausgewählt werden.

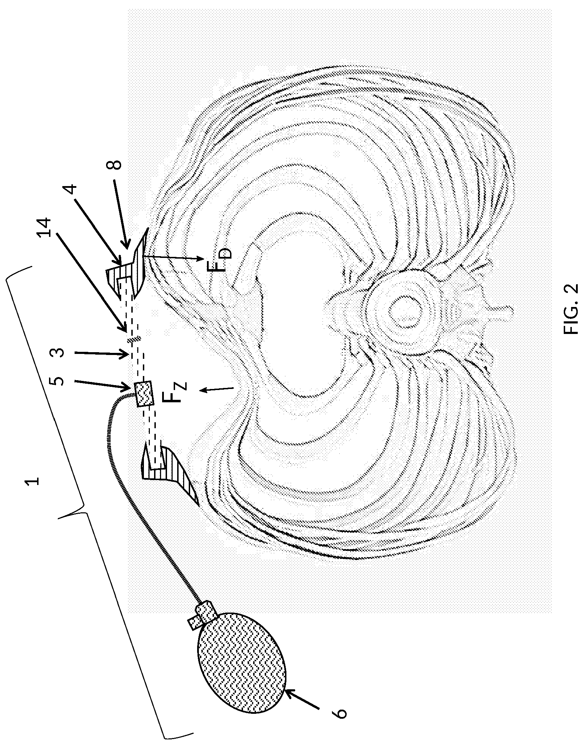

In Fig. 2 ist die Vorrichtung 1 entlang der horizontalen Schnittlinie II - II in Fig. 1 dargestellt. Im dargestellten Beispiel ist der Mittelteil 3 im Wesentlichen eben ausgebildet und mit dem Auflagerand 4 verbunden, insbesondere verklebt. Alternativ dazu kann der Mittelteil 3 und der Auflagerand 4 auch gemeinsam in einem Zwei- oder Mehrkomponenten-Spritzgussverfahren hergestellt werden. Anstelle einer ebenen Ausführung des Mittelteils 3 kann dieser auch in Richtung Brustkorb B gewölbt oder strukturiert ausgebildet sein (nicht dargestellt), wodurch dem Mittelteil eine höhere Stabilität bei gleichzeitig geringerer Materialdicke verliehen werden kann. Durch den Unterdruck zwischen Saugglocke 2 und Brustkorb B entsteht eine Zugkraft F_{Z} auf die eingewölbten Teile der Trichterbrust. Gleichzeitig wirkt über den Auflagerand 4 an den vorstehenden Rippenbögen eine Druckkraft F_{D}, wodurch der therapeutische Effekt verstärkt werden kann. Der Fortsatz 8 am Mittelteil 3 bzw. der darunter angeordnete Auflagerand 4 wird über den vorspringenden linken unteren Rippenbogen des Patienten P angeordnet, wodurch genau darauf die Druckkraft F_{D} wirkt und zusammen mit der Zugkraft F_{Z} auf die eingewölbten Teile der Trichterbrust zu einem besonders raschen und guten Therapieergebnis speziell bei dieser asymmetrischen Trichterbrustform führt.

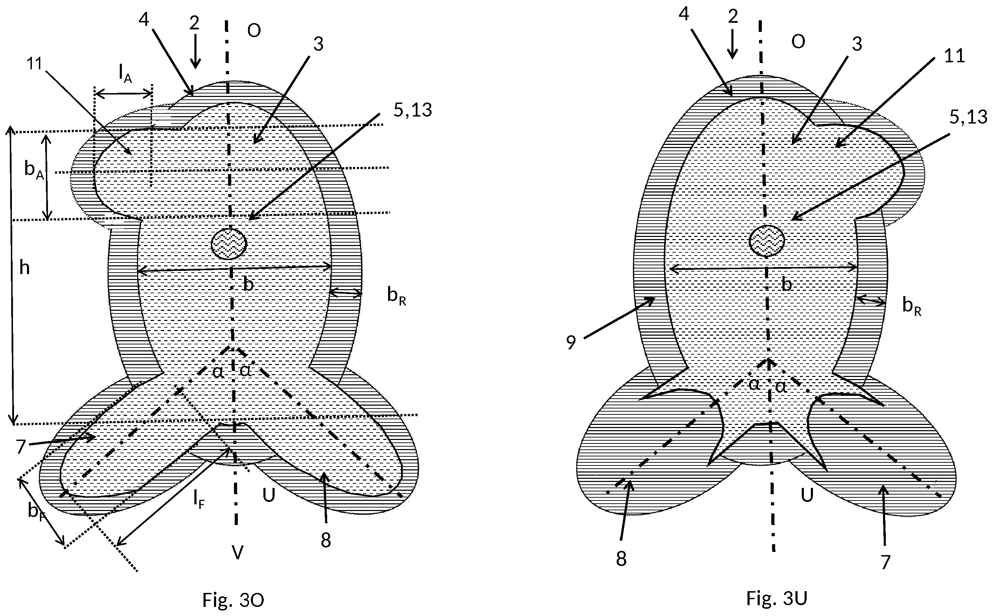

Fig. 3O und 3U zeigen schematische Ansichten auf eine weitere Ausführungsform einer Vorrichtung 1 zur nichtinvasiven Trichterbrustkorrektur von oben und unten. In der dargestellten Ausführungsform weist der Mittelteil 3 an der Unterseite U beiderseits lateral und schräg nach unten weisende Fortsätze 7, 8 und an der Oberseite O eine seitliche Ausbuchtung 11 auf. Die beiden Fortsätze 7, 8 sind vorzugsweise in einem Winkel α von 25° bis 60° zu einer in der Gebrauchslage der Saugglocke 2 vertikalen Richtung V angeordnet. Die Ausbuchtung 11 ist im Wesentlichen senkrecht zur vertikalen Richtung V, also in der Gebrauchslage im Wesentlichen horizontal angeordnet. Die Höhe h und Breite b des Mittelteils 3 der Saugglocke 2 kann an den Patienten P individuell angepasst werden. Beispielsweise haben sich Werte für die Höhe h zwischen 15 cm und 25 cm und für die Breite b zwischen 10 cm und 20 cm in den drei Größen (klein, mittel, groß) als geeignet herausgestellt. Die Länge l_{F} und Breite b_{F} der Fortsätze 7, 8 und die Länge l_{A} und Breite b_{A} der seitlichen Ausbuchtung 11 wird entsprechend an die Dimensionen des Mittelteils 3 angepasst und kann beispielsweise einen Bruchteil der Höhe h oder Breite b des Mittelteils 3 betragen.

Der Auflagerand 4 aus gewebeverträglichem Kunststoff weist eine bestimmte Höhe von beispielsweise 5 cm auf und verjüngt sich im Randbereich. Bei vollem Unterdruck erweitert sich der äußere Radius der abdichtenden Lippe des Auflagerandes 4. Nach Aufheben des Unterdrucks verkleinert sich der äußere Radius der Lippe und nimmt damit seine ursprüngliche Form an. Der Auflagerand 4 weist eine Breite b_{R}, beispielsweise 3 cm bis 5 cm, auf. Im Bereich des zumindest einen Fortsatzes 7, 8 und allenfalls auch im Bereich der zumindest einen seitlichen Ausbuchtung 11 weist der Auflagerand eine Verbreiterung mit einer gegenüber der übrigen Breite b_{R} größeren Breite, beispielsweise von 4 cm bis 8 cm auf. Dadurch kann die Druckkraft F_{D} auf die vorspringenden Teile des Brustkorbs B bei der Anwendung der Vorrichtung 1 besser verteilt werden. Entsprechend der Ansicht auf die Saugglocke 2 von unten in Fig. 3U sind die Auflageränder 4 unter den Fortsätzen 7, 8 in Form von ovalen Teilen verbreitert ausgeführt, wodurch sich eine bessere Kraftverteilung ergibt.

Die Materialien des Mittelteils 3 und des Auflagerandes 4 der Saugglocke 2 sollten antiallergisch, medizinisch zu reinigen und umfassend desinfizierbar sein. Die Mittelteile 3 sind vorzugsweise aus bruchsicherem und durchsichtigem Kunststoff in einer Steifigkeit ausgeführt, dass bei angewendeten Drucken keine relevante Verbiegung zustande kommt.

Die Figuren 4A bis 4J zeigen verschiedene Ausführungsformen der Vorrichtung 1 zur nichtinvasiven Trichterbrustkorrektur, welche die meisten Trichterbrustformen abdecken.

Die Ausführungsform der Vorrichtung 1 gemäß Fig. 4A weist einen linken Fortsatz 8 an der Unterseite U des Mittelteils 3 auf.

Diese Form kann besonders geeignet bei einer Trichterbrust mit zusätzlich vorspringendem linken unteren Rippenbogen angewendet werden. Dadurch wird einerseits in dem eingesunkenen Bereich (median bzw. leicht paramedian) der Unterdruck bzw. die Zugkraft und gleichzeitig auf den vorspringenden deformierten linken unteren Rippenbogen ein Überdruck bzw. eine Druckkraft ausgeübt.

Die Variante der Vorrichtung 1 gemäß Fig. 4B weist einen rechten Fortsatz 7 an der Unterseite U des Mittelteils 3 auf. Diese Form ist besonders geeignet bei einer Trichterbrust mit zusätzlich vorspringendem rechten unteren Rippenbogen.

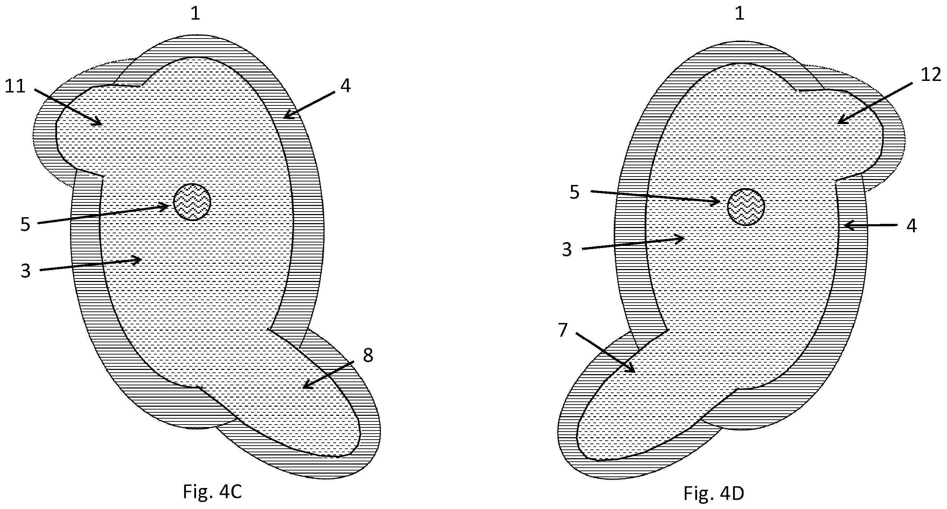

Bei der Variante der Vorrichtung 1 gemäß Fig. 4C ist zusätzlich zu der Variante gemäß Fig. 4A an der Oberseite O des Mittelteils 3 eine nach rechts weisende seitliche Ausbuchtung 11 angeordnet. Diese Form findet Anwendung bei einer Trichterbrust mit zusätzlich vorspringendem linken unteren Rippenbogen und eingesunkenem Brustkorb paramedian im rechten Oberfeld des Brustkorbs **B.** Dadurch wird einerseits in den eingesunkenen Bereichen (median bzw. leicht paramedian und im rechten Brustkorboberfeld) ein Unterdruck und gleichzeitig auf den vorspringenden deformierten linken unteren Rippenbogen ein Überdruck ausgeübt werden.

Die Variante der Vorrichtung 1 gemäß Fig. 4D ist spiegelbildlich zu der Ausführungsform gemäß 4C ausgebildet und weist an der Unterseite U des Mittelteils einen nach rechts unten weisenden Fortsatz 7 und an der Oberseite O des Mittelteils 3 eine nach links weisende seitliche Ausbuchtung 12 auf.

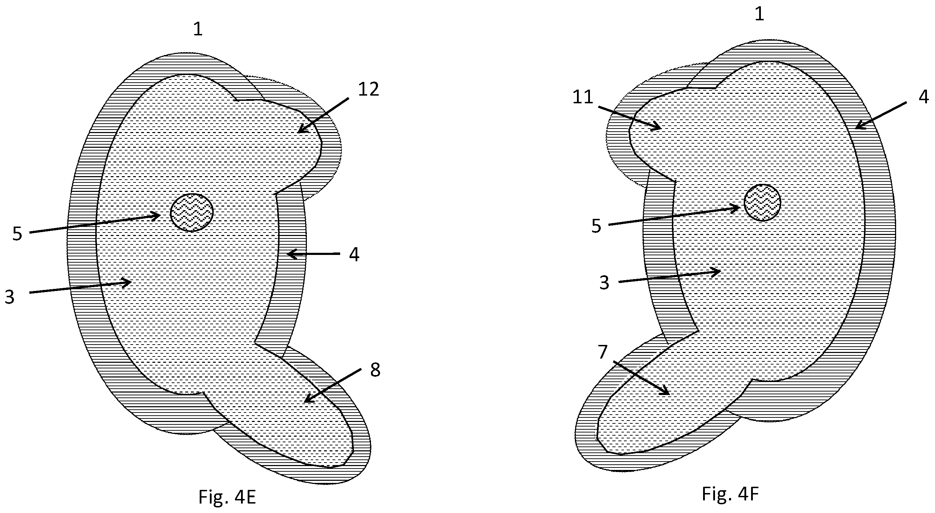

Die Ausführungsform der Vorrichtung 1 zur nichtinvasiven Trichterbrustkorrektur gemäß Fig. 4E zeigt einen nach links unten weisenden Fortsatz 8 und eine seitlich nach rechts weisende Ausbuchtung 12 auf. Diese Form findet Anwendung bei einer Trichterbrust mit zusätzlich vorspringendem linken unteren Rippenbogen und eingesunkenem Brustkorb paramedian im linken Brustkorboberfeld. Hier soll einerseits in den eingesunkenen Bereichen (median bzw. leicht paramedian und im linken Brustkorboberfeld) der Unterdruck und gleichzeitig auf den vorspringenden deformierten linken unteren Rippenbogen ein Überdruck angewendet werden.

Die Variante gemäß Fig. 4F ist spiegelbildlich zur Variante gemäß Fig. 4E angeordnet.

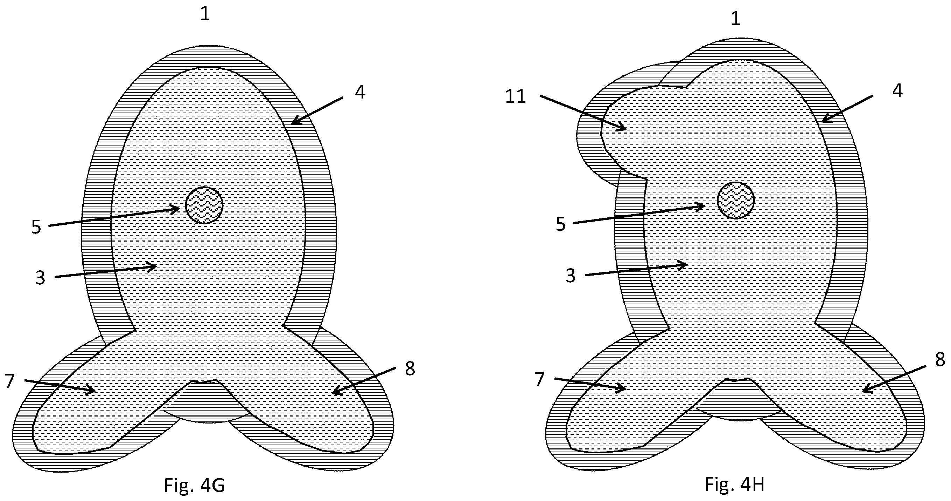

Die Ausführungsform der Vorrichtung 1 gemäß Fig. 4G zeigt an der Unterseite U des Mittelteils 3 beiderseits schräg nach unten weisende Fortsätze 7, 8 auf. Diese Form findet Anwendung bei einer Trichterbrust mit zusätzlich beidseits vorspringenden unterer Rippenbögen. Hier soll einerseits in den eingesunkenen Bereichen (median bzw. leicht paramedian) der Unterdruck und gleichzeitig auf den vorspringenden deformierten beidseitigen unteren Rippenbogen ein Überdruck angewendet werden.

Bei der Variante gemäß Fig. 4H ist zusätzlich zur Variante gemäß Fig. 4G an der Oberseite O des Mittelteils 3 eine seitlich nach rechts weisende Ausbuchtung 11 angeordnet, wodurch sich die Form bei einer Trichterbrust mit beidseits vorspringenden unteren Rippenbögen und zusätzlich eingesunkenem Brustkorb paramedian im rechten Bereich des Brustkorbs B besonders eignet.

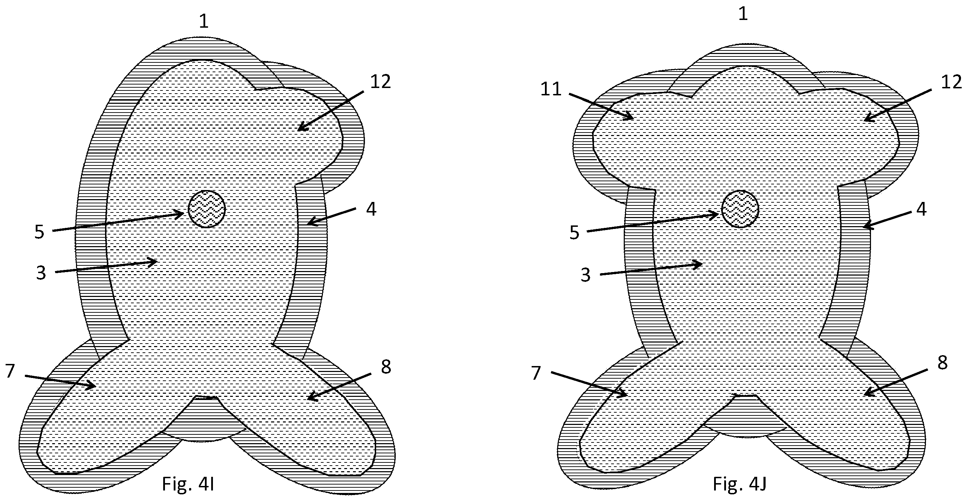

Figur 4I zeigt eine Form der Vorrichtung 1, wobei zusätzlich zur Variante gemäß Fig. 4G an der Oberseite O des Mittelteils 3 eine seitlich nach links weisende Ausbuchtung 12 angeordnet ist. Diese Form eignet sich besonders zur Anwendung bei einer Trichterbrust mit beidseits vorspringenden unteren Rippenbögen und zusätzlich eingesunkenem Brustkorb paramedian im linken Bereich des Brustkorbs B.

Schließlich zeigt die Ausführungsvariante der Vorrichtung 1 gemäß Fig. 4J eine Kombination der Varianten gemäß Fig. 4H und 4I mit beiderseitigen seitlichen Ausbuchtungen 11, 12 an der Oberseite O des Mittelteils 3.

Diese unterschiedlich geformten Vorrichtungen 1 bzw. Mittelteile 3 gemäß den Fig. 4A bis 4J können in drei verschiedene Dimensionen (klein, mittel, groß) vorgefertigt werden, um den verschiedenen Brustkorbgrößen gerecht zu werden.

## Patentansprüche

1. Vorrichtung (1) zur nichtinvasiven Trichterbrustkorrektur, mit einer Saugglocke (2) zum Aufsetzen auf den Brustkorb (B) eines Patienten (P), wobei die Saugglocke (2) einen Mittelteil (3) und einen daran anschließenden, umlaufenden Auflagerand (4) aus weichem Material zur Auflage auf dem Brustkorb (B) sowie einen Anschluss (5) zur Verbindung mit einer Luftpumpe (6) aufweist, **dadurch gekennzeichnet, dass** der Mittelteil (3) an seiner Unterseite (U) in der Gebrauchslage der Saugglocke (2) im Bereich der unteren Rippenbögen zumindest einen lateral und schräg nach unten weisenden Fortsatz (7, 8) aufweist, und dass der Auflagerand (4) im Bereich des zumindest einen Fortsatzes (7, 8) eine Verbreiterung gegenüber der Breite (b_{R}) des restlichen Auflagerandes (4) aufweist, sodass ein Druck (F_{D}) auf einen linken oder rechten unteren Rippenbogen ausübbar ist.

2. Vorrichtung (1) nach Anspruch 1, **dadurch gekennzeichnet, dass** der Mittelteil (3) an seiner Unterseite (U) in der Gebrauchslage der Saugglocke (2) im Bereich der unteren Rippenbögen beiderseits einen lateral und schräg nach unten weisenden Fortsatz (7, 8) aufweist, sodass ein Druck (F_{D}) auf beidseitige untere Rippenbögen ausübbar ist.

3. Vorrichtung (1) nach Anspruch 1 oder 2, **dadurch gekennzeichnet, dass** der zumindest eine Fortsatz (7, 8) in einem Winkel (α) von 25° bis 60° zu einer in der Gebrauchslage der Saugglocke (2) vertikalen Richtung (V) angeordnet ist.

4. Vorrichtung (1) nach einem der Ansprüche 1 bis 3, **dadurch gekennzeichnet, dass** der Mittelteil (3) an seiner Oberseite (O) in der Gebrauchslage der Saugglocke (2) im oberen Brustkorbbereich zumindest eine seitliche Ausbuchtung (11, 12) aufweist, sodass eingesunkene Bereiche im rechten und/oder linken Bereich des Brustkorb-Oberfeldes überdeckbar sind.

5. Vorrichtung (1) nach Anspruch 4, **dadurch gekennzeichnet, dass** der Auflagerand (4) im Bereich der zumindest einen seitlichen Ausbuchtung (11, 12) eine Verbreiterung gegenüber der Breite (b_{R}) des restlichen Auflagerandes (4) aufweist.

6. Vorrichtung (1) nach einem der Ansprüche 1 bis 5, **dadurch gekennzeichnet, dass** der Anschluss (5) zur Verbindung mit der Luftpumpe (6) ein Ventil (13) aufweist.

7. Vorrichtung (1) nach einem der Ansprüche 1 bis 6, **dadurch gekennzeichnet, dass** am Mittelteil (3) eine Vorrichtung (14) zur Begrenzung des Unterdrucks zwischen Saugglocke (2) und Brustkorb (B) des Patienten (P) bei der Anwendung der Saugglocke (2), vorzugsweise ein Sicherheitsventil, angeordnet ist.

8. Vorrichtung (1) nach einem der Ansprüche 1 bis 7, **dadurch gekennzeichnet, dass** der Mittelteil (3) im Wesentlichen eben ausgebildet ist.

9. Vorrichtung (1) nach einem der Ansprüche 1 bis 7, **dadurch gekennzeichnet, dass** der Mittelteil (3) in Richtung Brustkorb (B) gewölbt oder strukturiert ausgebildet ist.

10. Vorrichtung (1) nach einem der Ansprüche 1 bis 9, **dadurch gekennzeichnet, dass** der Mittelteil (3) zumindest teilweise aus transparentem Material besteht.

11. Vorrichtung (1) nach einem der Ansprüche 1 bis 10, **dadurch gekennzeichnet, dass** der Mittelteil (3) und der Auflagerand (4) gemeinsam hergestellt ist, vorzugsweise in einem Zweikomponenten-Spritzgussverfahren.

12. Vorrichtung (1) nach einem der Ansprüche 1 bis 10, **dadurch gekennzeichnet, dass** der Mittelteil (3) und der Auflagerand (4), vorzugsweise fest, miteinander verbunden sind.

## Claims

1. Device (1) for non-invasive correction of pectus excavatum, comprising a suction cup (2) for placement on the thorax (B) of a patient (P), wherein the suction cup (2) comprises a central section (3) and a surrounding bearing edge (4) made of soft material connected thereto for placement on the thorax (B), as well as a connector (5) for connection to an air pump (6), **characterised in that** the central section (3) has, on its underside (U) in the position of use of the suction cup (2), in the area of the lower costal arches, at least one laterally and obliquely downwardly oriented projection (7, 8), and **in that** the bearing edge (4) in the region of the at least one projection (7, 8) has a widening compared to the width (b_{R}) of the remaining bearing edge (4), so that a force (F_{D}) can be applied to a left or right lower costal arch.

2. Device (1) according to claim 1, **characterised in that** the central section (3) comprises, on its bottom side (U) in the position of use of the suction cup (2), in the area of the lower costal arches on both sides, a laterally and obliquely downwardly oriented projection (7, 8), so that a force (F_{D}) can be applied to the lower costal arches on both sides.

3. Device (1) according to claim 1 or 2, **characterised in that** the at least one projection (7, 8) is arranged at an angle (α) of 25° to 60° to a vertical direction (V) in the position of use of the suction cup (2).

4. Device (1) according to any one of claims 1 to 3, **characterised in that** the central section (3) comprises, on its top side (O) in the position of use of the suction cup (2), in the upper thoracic region, at least one lateral bulge (11, 12), so that sunken areas in the right and/or left region of the upper thorax can be covered.

5. Device (1) according to claim 4, **characterised in that** the bearing edge (4) has, in the area of the at least one lateral bulge (11, 12), a widening compared to the width (b_{R}) of the remaining bearing edge (4).

6. Device (1) according to any one of claims 1 to 5, **characterised in that** the connector (5) for connection to the air pump (6) comprises a valve (13).

7. Device (1) according to any one of claims 1 to 6, **characterised in that** a device (14) for limiting the negative pressure between suction cup (2) and thorax (B) of the patient (P) during use of the suction cup (2), preferably a safety valve, is arranged on the central section (3).

8. Device (1) according to any one of claims 1 to 7, **characterised in that** the central section (3) is formed substantially planar.

9. Device (1) according to any one of claims 1 to 7, **characterised in that** the central section (3) is formed as curved or structured in the direction of the thorax (B).

10. Device (1) according to any one of claims 1 to 9, **characterised in that** the central section (3) consists at least partially of transparent material.

11. Device (1) according to any one of claims 1 to 10, **characterised in that** the central section (3) and the bearing edge (4) are formed jointly, preferably in a two-component injection moulding process.

12. Device (1) according to any one of claims 1 to 10, **characterised in that** the central section (3) and the bearing edge (4) are preferably permanently connected to each other.

## Revendications

1. Dispositif (1) pour la correction non invasive du thorax en entonnoir, comprenant une ventouse thoracique (2) destinée à être placée sur le thorax (B) d'un patient (P), dans lequel la ventouse thoracique (2) comprend une partie centrale (3) et un bord d'appui périphérique (4) en matériau souple qui lui est adjacent pour l'appui sur le thorax (B), ainsi qu'un raccord (5) pour la connexion à une pompe à air (6), **caractérisé en ce que** la partie centrale (3) présente, sur sa face inférieure (U) dans la position d'utilisation de la ventouse thoracique (2), au moins un prolongement (7, 8) orienté latéralement et obliquement vers le bas dans la région des arcs costaux inférieurs, et **en ce que** le bord d'appui (4) présente, dans la région du au moins un prolongement (7, 8), un élargissement par rapport à la largeur (b_{R}) du reste du bord d'appui (4), de sorte qu'une pression (F_{D}) peut être exercée sur un arc costal inférieur gauche ou droit.

2. Dispositif (1) selon la revendication 1, **caractérisé en ce que** la partie centrale (3) présente, sur sa face inférieure (U) dans la position d'utilisation de la ventouse thoracique (2), un prolongement (7, 8) orienté latéralement et obliquement vers le bas de chaque côté dans la région des arcs costaux inférieurs, de sorte qu'une pression (F_{D}) peut être exercée sur les arcs costaux inférieurs des deux côtés.

3. Dispositif (1) selon la revendication 1 ou 2, **caractérisé en ce que** l'au moins un prolongement (7, 8) est disposé à un angle (α) de 25° à 60° par rapport à une direction verticale (V) dans la position d'utilisation de la ventouse thoracique (2).

4. Dispositif (1) selon l'une des revendications 1 à 3, **caractérisé en ce que** la partie centrale (3) présente, sur sa face supérieure (O) dans la position d'utilisation de la ventouse thoracique (2), dans la région supérieure du thorax au moins un renflement latéral (11, 12), de sorte que des zones affaissées dans la région droite et/ou gauche de la partie supérieure du thorax peuvent être couvertes.

5. Dispositif (1) selon la revendication 4, **caractérisé en ce que** le bord d'appui (4) présente, dans la région d'au moins un renflement latéral (11, 12), un élargissement par rapport à la largeur (b_{R}) du reste du bord d'appui (4).

6. Dispositif (1) selon l'une des revendications 1 à 5, **caractérisé en ce que** le raccord (5) pour la connexion à la pompe à air (6) comprend une valve (13).

7. Dispositif (1) selon l'une des revendications 1 à 6, **caractérisé en ce qu'**un dispositif (14) est disposé sur la partie centrale (3) pour limiter la dépression entre la ventouse thoracique (2) et le thorax (B) du patient (P) lors de l'utilisation de la ventouse thoracique (2), de préférence une soupape de sécurité.

8. Dispositif (1) selon l'une des revendications 1 à 7, **caractérisé en ce que** la partie centrale (3) est essentiellement plane.

9. Dispositif (1) selon l'une des revendications 1 à 7, **caractérisé en ce que** la partie centrale (3) est bombée ou structurée en direction du thorax (B).

10. Dispositif (1) selon l'une des revendications 1 à 9, **caractérisé en ce que** la partie centrale (3) est constituée, au moins partiellement, d'un matériau transparent.

11. Dispositif (1) selon l'une des revendications 1 à 10, **caractérisé en ce que** la partie centrale (3) et le bord d'appui (4) sont fabriqués ensemble, de préférence dans un procédé de moulage par injection à deux composants.

12. Dispositif (1) selon l'une des revendications 1 à 10, **caractérisé en ce que** la partie centrale (3) et le bord d'appui (4) sont reliés l'un à l'autre, de préférence de manière fixe.
